# EUROPEAN PATENT APPLICATION

(11) **EP 4 242 301 A1**
(43) Date of publication of application: **13.09.2023**
(21) Application number: 21888290.0
(22) Date of filing: 09.09.2021
(51) Int. Cl.: C12N 9/10, C12N 15/54, C12P 13/04

(54) **COMPOUND ENZYME AND APPLICATION THEREOF IN PREPARATION OF L-ERGOTHIONEINE**

(30) Priority: 03.11.2020 CN 202011209524
(71) Applicant: Shenzhen Readline Biotech Co., Ltd., Shenzhen, Guangdong 518052 (CN)
(72) Inventor: HUANG, Mingru, Shenzhen, Guangdong 518052 (CN); PAN, Junfeng, Shenzhen, Guangdong 518052 (CN); LIU, Jian, Shenzhen, Guangdong 518052 (CN)
(74) Representative: Germain Maureau
(86) International application number: PCT/CN2021/117379
(87) International publication number: WO 2022/095591

(57) **Abstract**

A compound enzyme, comprising L-histidine methylase, halogen methyltransferase and trimethylhistidine sulfurylase. L-histidine is catalyzed by L-histidine methylase and halogen methyltransferase to obtain trimethylhistidine, and then is catalyzed by trimethylhistidine sulfurase to obtain the L-ergothioneine. The method can realize conversion from L-histidine to L-ergothioneine only in two steps. Moreover, SAM enzyme is regenerated, such that the usage amount of the SAM enzyme is greatly reduced. Therefore, the method greatly reduces the raw material cost. Moreover, the method is high in reaction concentration (about 30 g/L), simple in process and good in industrial production prospect.

## Description

This application claims the priority of Chinese Patent Application No. 202011209524.3, filed with the China National Intellectual Property Administration on November 03, 2020, and titled with "COMPOUND ENZYME AND APPLICATION THEREOF IN PREPARATION OF L-ERGOTHIONEINE", which is hereby incorporated by reference in its entirety.

### FIELD

The present disclosure relates to the technical field of bioengineering, in particular to an enzyme composition and use thereof in the production of L-ergothioneine.

### BACKGROUND

L-ergothioneine, also known as thiolhistidinebetaine, is a sulfur-containing L-histidine derivative, widely found in human diets such as mushrooms, black beans, red meat and oats. In general, it is acquired and retained in most cells and tissues in human body through highly specific L-ergothioneine transporters. L-ergothioneine is a natural antioxidant that can effectively remove active oxygen and nitrogen in the body to protect cells from oxidation, apoptosis and damage. In addition, a large number of studies have shown that L-ergothioneine can also protect the skin from UV-radiation, and can effectively reduce the risk of diseases such as eye disease, inflammatory bowel disease, and liver fibrosis. Therefore, dietary supplementation of L-ergothioneine is becoming a potential option for preventing the above diseases. However, the high price has greatly restricted the further promotion and application of L-ergothioneine, so it is very important to develop a simple method for large-scale production of L-ergothioneine.

Although L-ergothioneine has a small molecular weight (C₉H₁₅N₃O₂S, 229.3), it contains trimethylammonium and sulfurized nitrogen heterocyclic functional groups which determine the specificity of the compound's function and production. At present, methods for producing L-ergothioneine include chemical synthesis, fermentation and enzymatic catalysis.

The chemical synthesis method uses L-histidine methyl ester as a starting material and requires multiple selective protection and deprotection of functional groups to indirectly realize the sulfuration of the nitrogen heterocycle and the trimethylation of the amino group (as shown in FIG. 2, refer to Patent No. US7767826B2). The whole production process is very cumbersome, and has low final yield and high environmental cost.

The research on the fermentation production of L-ergothioneine has a long history. For example, Pinghua Liu in the United States implanted two L-ergothioneine synthesis genes, Egt-1 and EgtE, into *Escherichia coli,* and then used L-histidine or L-hercynine as a raw material for fermentation to produce L-ergothioneine (refer to Patent No. WO 2014/100752 A1). Steven A. van der Hoek in the United Kingdom modified *Saccharomyces cerevisiae* and used common yeast culture medium for fermentation to produce L-ergothioneine (reference: Steven A. van der Hoek et al, Front. Bioeng. Biotech. 2019, 7, 262). However, the low yield of fermentation production of L-ergothioneine (the highest concentration achieved according to reports is close to gram per liter) makes it hard to realize large-scale production currently.

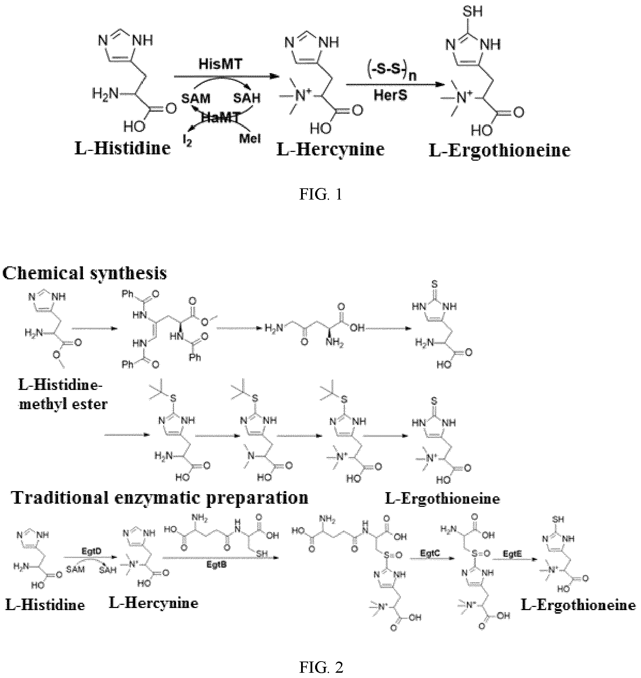

The *in vitro* enzymatic catalysis production of L-ergothioneine also has related reports, which adopted the L-ergothioneine Egt series synthetase in the organism for catalysis (as shown in FIG. 2, reference: Florian P. Seebeck, JACS, 2010, 132, 6632-6633). However, the long route, the low conversion efficiency of EgtD enzyme and EgtB enzyme, and the large amount of S-adenosylmethionine consumed cause the cost of enzymatic synthesis production of L-ergothioneine to remain high.

It can be seen that it is still necessary to further develop a method for industrialized production of L-ergothioneine with low cost and high yield at present.

### SUMMARY

In view of this, the technical problem to be solved by the present disclosure is to provide an enzyme composition and use thereof in the production of L-ergothioneine, through which L-ergothioneine can be obtained by a two-step reaction.

The present disclosure provides an enzyme composition comprising L-histidine methyltransferase, halogen methyltransferase and L-hercynine sulfurylase.

In the enzyme composition of the present disclosure, the L-histidine methyltransferase is derived from *Mycobacterium smegmatismc* or *Chlorobium limicola;* the halogen methyltransferase is derived from *Chloracidobacterium thermophilum* or *Mycobacterium smegmatis;* and the L-hercynine sulfurylase is derived from *Escherichia coli* or *Chlorobium limicola.*

In an embodiment of the present disclosure, the L-histidine methyltransferase has an amino acid sequence set forth in SEQ ID NO: 1 or 2, the halogen methyltransferase has an amino acid sequence set forth in SEQ ID NO: 3 or 4, and the L-hercynine sulfurylase has an amino acid sequence set forth in SEQ ID NO:5 or 6.

In the present disclosure, the L-histidine methyltransferase derived from *Mycobacterium smegmatismc* is referred to as MsHisMT, and the L-histidine methyltransferase derived from *Chlorobium limicola* is referred to as ClHisMT. The halogen methyltransferase derived from *Chloracidobacterium thermophilum* is referred to as CtHaMT, and the halogen methyltransferase derived from *Mycobacterium smegmatis* is referred to as MsHaMT. The L-hercynine sulfurylase derived from *Escherichia coli* is referred to as EcHerS, and the L-hercynine sulfurylase derived from *Chlorobium limicola* is referred to as ClHerS.

In some embodiments, the enzyme composition consists of MsHisMT, CtHaMT and EcHerS. In such embodiments, MsHisMT and CtHaMT are in a ratio of 1000U: 1500U.

In some other embodiments, the enzyme composition consists of ClHisMT, CtHaMT and ClHerS. In such embodiments, ClHisMT and CtHaMT are in a ratio of 800U:800U.

In some other embodiments, the enzyme composition consists of MsHisMT, MspHaMT and ClHerS. In such embodiments, MsHisMT and MspHaMT are in a ratio of 500U: 1000U.

The present disclosure further provides use of the enzyme composition of the present disclosure in the production of L-ergothionein by enzymatic catalysis.

The present disclosure further provides a method for producing L-ergothionein by enzymatic catalysis, comprising
catalyzing L-histidine by L-histidine methyltransferase and halogen methyltransferase to obtain L-hercynine; and catalyzing L-hercynine by L-hercynine sulfurylase to obtain L-ergothioneine.

In the step of obtaining L-hercynine from L-histidine, halogen methyltransferase HaMT is added, which can rapidly convert S-adenosylhomocysteine (SAH) into S-adenosylmethionine (SAM) using iodomethane. Therefore, in this step, HaMT enzyme and HisMT enzyme are both used to regenerate S-adenosylmethionine using L-histidine methyltransferase HisMT and halogen methyltransferase HaMT to convert the raw material L-histidine into L-hercynine at once.

In the method of the present disclosure, L-hercynine is prepared by adding L-histidine, S-adenosylmethionine, L-histidine methyltransferase and halogen methyltransferase to a buffer solution, then adding iodomethane dropwise, and stirring at pH7.0-9.0 for reaction to obtain L-hercynine.

In some embodiments, the buffer solution is a phosphate buffer solution at 100 mmol/L with a pH of 8.0;
the L-histidine added to the reaction system has a concentration of 100 mmol/L-200 mmol/L, the S-adenosylmethionine has a concentration of 0.8 mmol/L-1.2 mmol/L, the L-histidine methyltransferase has a concentration of 500 U/L-1000 U/L, and the halogen methyltransferase has a concentration of 800 U/L-1500 U/L; and
the dropwise added iodomethane and S-adenosylmethionine are in a molar ratio of (360-720): 1.

The dropwise addition lasts for 4 h, and the stirring is maintained for 4 h after the dropwise addition.

After L-hercynine is obtained, the reaction solution is extracted with ethyl acetate. The aqueous phase is subjected to ultrafiltration at a molecular weight of 1000-5000 to obtain a filtrate as a solution containing L-hercynine.

Wherein, the extraction removes iodomethane and iodine in the reaction solution and the ultrafiltration removes MsHisMT enzyme and CtHaMT enzyme in the reaction solution. The resulting solution containing L-hercynine is vacuumized with a diaphragm vacuum pump and supplemented with N₂ to remove O₂ from the reaction solution, and finally sealed under N₂ protection.

In the method of the present disclosure, the catalyzing of L-hercynine by L-hercynine sulfurylase to obtain L-ergothioneine comprises adding L-hercynine sulfurylase and potassium polysulfide for reaction in N₂ atmosphere to obtain L-ergothioneine. The reaction in N₂ atmosphere is carried out for 6 h.

In some embodiments, the L-hercynine sulfurylase is added to a concentration of 600 U/L-800 U/L, and the potassium polysulfide is added at an amount twice the equivalent of L-hercynine.

The obtained reaction solution containing L-ergothioneine is precipitated with ethanol, and then the supernatant is collected, concentrated, and loaded on an anion exchange column. After elution with water, concentration and drying, a crude product of L-ergothioneine is obtained. The crude product of L-ergothioneine is crystallized with ethanol/water solution to obtain a pure product of L-ergothioneine. The anion exchange column is Amberjet^{®} 4200, -OH. The precipitation with ethanol removes EcHerS enzyme and phosphate.

In some embodiments, the L-histidine methyltransferase is prepared by transforming a host in which S-adenosylhomocysteine nucleoside hydrolase mtnN gene is knocked out with a nucleic acid molecule encoding L-histidine methyltransferase, and performing induction and expression to obtain a bacterial solution containing the L-histidine methyltransferase;
the halogen methyltransferase is prepared by transforming a host in which S-adenosylhomocysteine nucleoside hydrolase mtnN gene is knocked out with a nucleic acid molecule encoding halogen methyltransferase, and performing induction and expression to obtain a bacterial solution containing the halogen methyltransferase; and
the L-hercynine sulfurylase is prepared by transforming a host in which S-adenosylhomocysteine nucleoside hydrolase mtnN gene is knocked out with a nucleic acid molecule encoding L-hercynine sulfurylase, and performing induction and expression to obtain a bacterial solution containing the L-hercynine sulfurylase.

The enzyme expressed and produced by the host mtnN gene will degrade SAH, resulting in the inability to regenerate SAM. Therefore, when constructing a recombinant host, a host without mtnN gene expression or a host in which mtnN gene is knocked out is selected. In some embodiments, the host cell of the recombinant host is *Escherichia coli.* Specifically, the host cell is *Escherichia coli* BL21-DE3.

The present disclosure further provides a nucleic acid molecule encoding L-histidine methyltransferase, selected from the group consisting of
I) a nucleic acid molecule having a nucleotide sequence set forth in SEQ ID NO: 7 or 8;
II) a nucleic acid molecule derived from I) by substitution, deletion or addition of one or more nucleotides;
III) a nucleic acid molecule having at least 85% homology with I) and encoding L-histidine methyltransferase; and
IV) a nucleic acid molecule partially or fully complementary to any one of I) to III).

The present disclosure further provides a nucleic acid molecule encoding halogen methyltransferase, selected from the group consisting of
i) a nucleic acid molecule having a nucleotide sequence set forth in SEQ ID NO: 9 or 10;
ii) a nucleic acid molecule derived from i) by substitution, deletion or addition of one or more nucleotides;
iii) a nucleic acid molecule having at least 85% homology with i) and encoding L-histidine methyltransferase; and
iv) a nucleic acid molecule partially or fully complementary to any one of i) to iii).

The present disclosure further provides a nucleic acid molecule encoding L-hercynine sulfurylase, selected from the group consisting of
a) a nucleic acid molecule having a nucleotide sequence set forth in SEQ ID NO: 11 or 12;
b) a nucleic acid molecule derived from a) by substitution, deletion or addition of one or more nucleotides;
c) a nucleic acid molecule having at least 85% homology with a) and encoding L-hercynine sulfurylase; and
d) a nucleic acid molecule partially or fully complementary to any one of a) to c).

The present disclosure further provides a recombinant vector, comprising the nucleic acid molecule encoding L-histidine methyltransferase and a backbone vector. In some embodiments, the backbone vector is pET28a.The nucleic acid fragment is inserted at a site of Nde I/Xho I.

Alternatively, the recombinant vector comprises the nucleic acid molecule encoding halogen methyltransferase and a backbone vector. In some embodiments, the backbone vector is pET28a. The nucleic acid fragment is inserted at a site of Nde I/Xho I.

Alternatively, the recombinant vector comprises the nucleic acid molecule encoding halogen methyltransferase and a backbone vector. In some embodiments, the backbone vector is pET28a. The nucleic acid fragment is inserted at a site of Nco I/Xho I.

The present disclosure further provides a recombinant host transformed or transfected with the recombinant vector, wherein in the host, S-adenosyl homocysteine nucleoside hydrolase mtnN gene is knocked out.

The present disclosure provides an enzyme composition, which comprises L-histidine methyltransferase, halogen methyltransferase and L-hercynine sulfurylase. L-histidine is catalyzed by L-histidine methyltransferase and halogen methyltransferase to obtain L-hercynine, which is then catalyzed by L-hercynine sulfurylase to obtain L-ergothioneine. The method can realize conversion from L-histidine to L-ergothioneine only in two steps. Moreover, the regeneration of SAM enzyme greatly reduces the amount of the SAM enzyme used. Therefore, the method greatly reduces the raw material cost. Meanwhile, the method has high product concentration (about 30 g/L), simple process and good industrial production prospects.

### BRIEF DESCRIPTION OF DRAWINGS

In order to more clearly illustrate the embodiments of the present disclosure or the technical solutions in the prior art, the following briefly introduces the drawings needed to be used in the description of the embodiments or the prior art.
FIG. 1 shows the route of enzymatic production of L-ergothionein in the present disclosure;
FIG. 2 shows the route of production of L-ergothioneine adopted on the market;

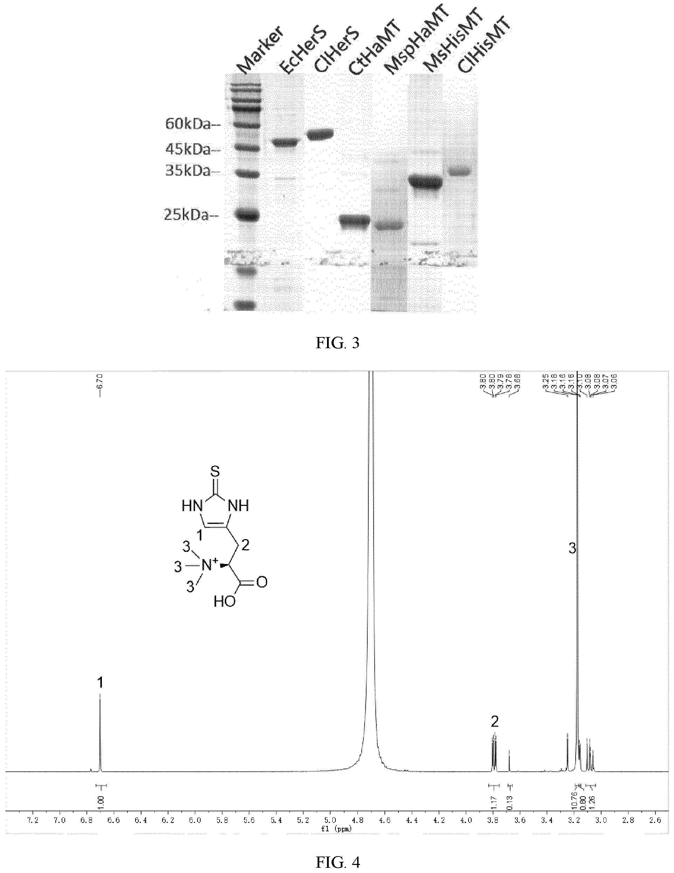

FIG. 3 shows the SDS-PAGE gel images of L-histidine methyltransferase HisMT, halogen methyltransferase HaMT and L-hercynine sulfurylase HerS in the examples;
FIG. 4 shows the nuclear-magnetic ¹H-NMR data of the finally purified L-ergothioneine in 600M Varian, D₂O solution.

### DETAILED DESCRIPTION

The present disclosure provides an enzyme composition and use thereof in the production of L-ergothioneine. Those skilled in the art can learn from the content herein and appropriately improve the process parameters for realization. In particular, it should be noted that all similar replacements and modifications are apparent to those skilled in the art, which are all considered to be included in the present disclosure. The method and use of the present disclosure have been described through preferred embodiments, and those skilled in the art can apparently make modifications or appropriate changes and combinations to the method and use herein without departing from the content, spirit and scope of the present disclosure to realize and apply the technology of the present disclosure.

Various L-ergothioneine synthetases exist widely in nature. Organisms generally convert L-histidine into L-hercynine using L-histidine methyltransferase (EgtD, HisMT, EC 2.1.1.44). Subsequently, in most aerobic microorganisms, L-hercynine is continuously converted into L-ergothioneine by L-hercynine oxidase (EgtB, EC 1.14.99.50), glutamate hydrolase (EgtC, EC 3.5.1.118) and sulfur oxygen lyase (EgtE, EC 4.4.1.36).

The present disclosure obtains a variety of L-histidine methyltransferase HisMT and L-hercynine sulfurylase HerS through screening and selects HisMT and HerS that are overexpressed and highly active in *Escherichia coli,* thereby effectively realizing the scaled-up production of L-hercynine sulfuration. Moreover, a halogen methyltransferase HaMT is introduced in the production process, and the enzyme can rapidly convert S-adenosylhomocysteine (SAH) into S-adenosylmethionine (SAM) using iodomethane. Therefore, HaMT enzyme and HisMT enzyme are both used to effectively realize the cyclic regeneration of the expensive coenzyme S-adenosylmethionine.

In order to achieve the above purpose of the present disclosure, the present disclosure provides the following technical solution: regenerating S-adenosylmethionine by utilizing L-histidine methyltransferase HisMT and halogen methyltransferase HaMT to convert the raw material L-histidine into L-hercynine at once; and then converting L-hercynine into L-ergothioneine by using L-hercynine sulfurylase HerS.

In some specific embodiments of the present disclosure, L-histidine methyltransferase (HisMT, EC 2.1.1.44), halogen methyltransferase (HaMT), and L-hercynine sulfurylase HerS belong to enzyme families of PF10017, PF05724, and PF00581, respectively, which may be derived from strains such as *Escherichia coli, Mycobacterium smegmatis, Chlorobium limicola, Chloracidobacterium thermophilum,* and *Methyllibium sp..*

In the present disclosure, the amino acid sequences of enzymes are derived from bacteria strains, and the nucleic acid sequences encoding the enzymes are codon-optimized. The optimization includes: codon usage preference, elimination of secondary structures (such as hairpin structures) that are not conducive to expression, changes in GC content, CpG dinucleotide content, secondary structure of mRNA, cryptic splice sites, early polyadenylation sites, internal ribosome entry site and ribosome-binding site, negative CpG islands, RNA instability regions, repeats (direct repeats, inverted repeats, etc.) and restriction sites that may affect cloning. In the present disclosure, the nucleic acid encoding the enzyme may be DNA, RNA, cDNA or PNA. In an embodiment of the present disclosure, the nucleic acid is in the form of DNA. Such DNA form includes cDNA, genomic DNA or synthetic DNA. The DNA may be single-stranded or double-stranded. The DNA may be either a coding strand or a non-coding strand.

The recombinant vector of the present disclosure comprises one or more regulatory sequences and coding sequences of the target protein. A regulatory sequence may include a promoter, an enhancer, a transcription termination signal, a polyadenylation sequence, an origin of replication, a nucleic acid restriction site, and a homologous recombination site operably linked to a nucleic acid sequence. The vector can also comprise a selective marker, such as a resistance protein marker, an amino acid screening marker or a green fluorescent protein.

The test materials used in the present disclosure are all commonly commercially available, which all can be purchased from the market, where the primer sequences used and the amino acid sequences and nucleic acid sequences of the enzymes are shown in Tables 1-3:

**Table 1**

| Primer name | Primer sequence |
|---|---|
| MsHisMT forward primer | GTGAAAGGACGC**CATATG**ACGCTCTCACTG |
| MsHisMT reverse primer | CACTGCTG**CTCGAG**CATCACCGCACCGCCAG |
| EcHerS forward primer | CTCAAAACTA**CCATGG**CGGGATTTTCGATG |
| EcHerS reverse primer | GACGCTGAAGTCG**CTCGAG**CGTCAGTAATTG |
| MsHisMT: Mycobacterium smegmatis-derived L-histidine methyltransferase | |
| ClHisMT: Chlorobium limicola-derived L-histidine methyltransferase | |
| CtHaMT: Chloracidobacterium thermophilum-derived halogen methyltransferase | |
| MspHaMT: Methylibium sp.-derived halogen methyltransferase | |
| ClHerS: Chlorobium limicola-derived L-hercynine sulfurylase | |
| EcHerS: Escherichia coli-derived L-hercynine sulfurylase | |
| CkHerS: Citrobacterkoseri-derived L-hercynine sulfurylase | |

**Table 2**

| Abbreviation | Sequence number | Amino acid sequence |
|---|---|---|
| MsHisMT | SEQ ID No. 1 | MTLSLANYLAADSAAEALRRDVRAGLTAAPKSLPPKWFY DAVGSDLFDQITRLPEYYPTRTEAQILRTRSAEIIAAAGAD TLVELGSGTSEKTRMLLDAMRDAELLRRFIPFDVDAGVL RSAGAAIGAEYPGIEIDAVCGDFEEHLGKIPHVGRRLVVFL GSTIGNLTPAPRAEFLSTLADTLQPGDSLLLGTDLVKDTG RLVRAYDDAAGVTAAFNRNVLAVVNRELSADFDLDAFE HVAKWNSDEERIEMWLRARTAQHVRVAALDLEVDFAAG EEMLTEVSCKFRPENVVAELAEAGLRQTHWWTDPAGDF GLSLAVR MAYSKTNLSELPLADIDNHLTEIGFDTTISEIITGLTANAKY IQSKYFYDKRGSALFEKITSLSEYYPSRTEKAIISQLPPALIE DLADIDIIELGCGDHSKISLLIRRIPAESVPGLRYFPIDISQTA LKQSIEDLRDLFPALKVKGILADYVHQMHLFPEERKRLFC FFGSTIGNLSREETLDFMQNMGTTMHPGDMLLVGMDRV KNIALLEKAYNDDQFITAMFNKNILRVINGLIKSDFNPDDF EHRAFYNADFNRIEMHLEATGNISVKSAFMPELIRIKKGET |
| ClHisMT | SEQ ID No. 2 | MAYSKTNLSELPLADIDNHLTEIGFDTTISEIITGLTANAKY IQSKYFYDKRGSALFEKITSLSEYYPSRTEKAIISQLPPALIE DLADIDIIELGCGDHSKISLLIRRIPAESVPGLRYFPIDISQTA LKQSIEDLRDLFPALKVKGILADYVHQMHLFPEERKRLFC FFGSTIGNLSREETLDFMQNMGTTMHPGDMLLVGMDRV KNIALLEKAYNDDQFITAMFNKNILRVINGLIKSDFNPDDF EHRAFYNADFNRIEMHLEATGNISVKSAFMPELIRIKKGET |
| | | IHTENSHKFEKADILLMGQHAGLAIKNIYSDKNELFSLAH YEKK |
| CtHaMT | SEQ ID No. 3 | MLGMDADTASFWEEKYRADLTAWDRGGVSPALEHWLA EGALKPGRILIPGCGYGHEVLALARRGFEVWGLDIALTPV RRLQEKLAQAGLTAHVVEGDVRTWQPEQPFDAVYEQTC LCALSPEDWPRYEAQLCRWLRPGGRLFALWMQTDRPGG PPYHCGLEAMRVLFALERWRWVEPPQRTVPHPTGFFEYA AILERLV |
| MspHaMT | SEQ ID No. 4 | MSGPDLNFWQQRFDAGQLPWDRGAPSPQLAAWLGDGSL KPGRIAVPGCGSGHEVVALARGGFSVTAIDYAPGAVRLT QGRLAAAGLAAEVVQADVLTWQPTAPLDAVYEQTCLCA LHPDHWVAYFARLHAWLRPGGTLALLAMQALRESAGQG LIEGPPYHVDVNALRALLPGDRWDWPRPPYARVPHPSST WQNWPSC |
| ClHerS | SEQ ID No. 5 | MQNKNFRAPQSEAIGILYKLIETGSKHKNMYDHTEITTDS LLALLGSEKVKIIDVRSADAYNGWRMRGEVRGGHIKGAK SLPAKWLTDPEWLNIVRFKQIRPEDAIVLYGYTPEECEQT ATRFKENGYNNVSVFHRFHPDWTGNDAFPMDRLEQYNR LVPAEWVNGLISGEEIPEYDNDTFIVCHAHYRNRDAYLSG HIPGATDMDTLALESPETWNRRTPEELKKALEEHGITAST TVVLYGKFMHPDNADEFPGSAAGHIGAIRLAFIMMYAGV EDVRVLNGGYQSWTDAGFAISKDDVPKTTVPEFGAPIPSR PEFAVDIDEAKEMLQSEDSDLVCVRSYPEYIGEVSGYNYI KKKGRIPGAIFAECGSDAYHMENYRNHDHTTREYHEIEDI WAKSGIIPKKHLAFYCGTGWRGSEAWFNALLMGWPRVS VYDGGWFEWSNDPENPYETGVPK |
| EcHerS | SEQ ID No. 6 | MSGFSMKRVSQMTALAMALGLACASSWAAELAKPLTLD QLQQQNGKAIDTRPSAFYNGWPQTLNGPSGHEPAALNLS ASWLDKMSTEQLNEWIKQHNLKADAPVALYGNDKDVD AVKTRLQKAGFTHISILSDALSEPSRLQKLPHFEQLVYPQ WLHNLQQGKEVTAKPAGDWKVIEAAWGAPKFYLISHIPG ADYIDTNEVESEPLWNKVSDEQLKAMLAKHGIRHDTTVI LYGRDVYAAARVAQIMLYAGVKDVRLLDGGWQTWSDA GLPVERGTPPKVKAEPDFGVKIPAQPQLMLDMEQARGLL HRQDASLVSIRSWPEFIGTTSGYSYIKPKGEIAGARWGHA GSDSTHMEDFHNPDGTMRSADDITAMWKAWNIKPDQQV SFYCGTGWRASETFMYARAMGWNNVSVYDGGWYEWSS DPKNPVATGERGPDSSK |

**Table 3**

| Abbreviation | Sequence number | Gene sequence of enzyme |
|---|---|---|
| MsHisMT | SEQ ID No. 1 | atgacgctctcactggccaactacctggcagccgactcggccgccgaagcactgcgccgt gacgtccgcgcgggcctcaccgcggcaccgaagagtctgccgcccaagtggttctacga cgccgtcggcagtgatctgttcgaccagatcacccggctccccgagtattaccccacccgc accgaggcgcagatcctgcggacccggtcggcggagatcatcgcggccgcgggtgccg acaccctggtggaactgggcagtggtacgtcggagaaaacccgcatgctgctcgacgcca tgcgcgacgccgagttgctgcgccgcttcatcccgttcgacgtcgacgcgggcgtgctgc gctcggccggggcggcaatcggcgcggagtaccccggtatcgagatcgacgcggtatgt ggcgatttcgaggaacatctgggcaagatcccgcatgtcggacggcggctcgtggtgttcc tggggtcgaccatcggcaacctgacacccgcgccccgcgcggagttcctcagtactctcg cggacacgctgcagccgggcgacagcctgctgctgggcaccgatctggtgaaggacacc ggccggttggtgcgcgcgtacgacgacgcggccggcgtcaccgcggcgttcaaccgca acgtgctggccgtggtgaaccgcgaactgtccgccgatttcgacctcgacgcgttcgagca tgtcgcgaagtggaactccgacgaggaacgcatcgagatgtggttgcgtgcccgcaccgc acagcatgtccgcgtcgcggcactggacctggaggtcgacttcgccgcgggtgaggaga tgctcaccgaggtgtcctgcaagttccgtcccgagaacgtcgtcgccgagctggcggaag ccggtctgcggcagacgcattggtggaccgatccggccggggatttcgggttgtc gctggcggtgcggtga |
| ClHisMT | SEQ ID No. 2 | atggcatactcgaagaccaatctttccgaactgccccttgcggacatagacaaccatttaact gagataggcttcgacactactatttcagagattattactgggttgactgctaatgcgaagtata ttcagtccaagtacttttatgataaacggggttctgctttatttgagaaaattacttcattatcaga gtactacccgagtagaacagagaaggctataatttcacaattaccgccagccttaatagaag atctggctgacatcgacattatcgagttgggctgcggagatcacagcaagatctcactgctt atacgccggatacccgccgaatctgtgcctggtttgcgctatttcccaattgatatctctcaaa cggctttaaaacaatcaatcgaggacttgagagacttatttccagccctgaaggttaaaggc atccttgcagattatgttcaccagatgcacctgttcccagaggaacgtaaacgcttgttttgttt ctttggttctacgataggcaatctgtcacgtgaagagacactggattttatgcagaacatggg cacaactatgcacccaggcgatatgttactggtcggtatggatcgtgtaaagaatatcgcatt actggagaaggcatataatgacgatcaattcataacggcaatgtttaacaaaaatatattgcg cgttataaacggattaataaaatctgacttcaatcctgacgatttcgagcaccgtgctttttataa tgcggactttaacagaatcgaaatgcaccttgaagctaccggtaatatctcggtgaagagcg cattcatgccagaactgatacggattaaaaaaggagaaactatccacacagagaacagtca caaatttgagaaagctgatatattgttaatgggacagcatgcagggttagcgatcaagaaca tctattccgacaagaacgaactttttagcctggcccattatgagaagaagtga |
| CtHaMT | SEQ ID No. 3 | atgctgggcatggacgctgatactgcgagcttctgggaagagaaatatcgcgccgacttaa ccgcatgggatcggggcggtgtatctcccgctctggaacattggttggccgagggtgcgtt |
| | | gaaacccggtcgcattttgataccgggctgcgggtatggccacgaagtcttagctttagctc ggcgcggatttgaggtttgggggcttgacatagcactgacacccgttcgtcggcttcaaga aaagttggcacaggctggtcttacggcacacgtagtcgagggcgacgtgcgtacttggca acccgaacaaccgttcgatgcagtatatgaacaaacatgcttatgcgcccttagccccgaag attggccacggtacgaagcccaattgtgccgttggcttagacctggcggcagattattcgca ctttggatgcagacagaccggccaggcggaccgccataccattgtgggttggaagcaatg cgtgttttatttgcgcttgaaagatggcgttgggtcgaaccgccccagagaacagtgccaca ccctacaggtttcttcgagtatgccgcgatccttgaacggttggtctga |
| MspHaMT | SEQ ID No. 4 | atgagtggacccgacttaaatttctggcagcaacgttttgacaccggccaacttccgtggga ccgtggcgcaccttcgccacaactggcggcttggttgggtgatgggtcattagcgccaggt cgcattgccgtaccggggtgcggttcaggacatgaagttgttgcgttagcccgcggtgggt tctcagtaacagccattgattacgcccccggagcagtgcgcttaacccaaggccgcttggct gcggcggggttagcggcggaagtcgtgcaggctgacgtacttacgtggcagccaacggc tccgcttgacgctgtgtacgagcaaacttgtctgtgtgcattgcatcccgaccattgggtggc atacgcagctagattacacgcttggcttagaccaggaggtactttggcattgttggcaatgca ggcactgcgtgagggcgctggacagggcttgatagaagggcctccttaccatgttgacgtt aacgctcttcgcgcgctgctgccgggggaccgttgggactggccacgtccaccgtatgcg cgcgttcctcacccctcttctacatgggccgagttggcaatagtcctgactcggcgttga |
| ClHerS | SEQ ID No. 5 | atgcagaataagaattttcgtgcaccccagtcagaggccataggcatcttgtataaacttattg aaactggatcgaagcacaaaaacatgtacgaccacacagagatcacaacagacagtctgtt ggcacttttgggttcggaaaaggtgaagataattgacgttcgctcggctgacgcgtataacg gttggcggatgcgtggtgaagtacgtggaggccacatcaaaggtgcaaagtcgttgccag cgaagtggctgacagatccggaatggcttaacattgttcgtttcaaacagatacgtcccgag gacgccatcgtcctttatggttatacgcccgaggagtgcgaacaaactgccactagattcaa agagaatggttataataatgtgagcgtatttcacagattccacccggactggactgggaacg atgctttcccaatggatagactggagcaatataatagacttgtacccgccgaatgggttaacg gtttgataagcggggaagagatcccagagtacgacaatgacaccttcatcgtgtgtcatgca cattatcggaacagagatgcgtatcttagcggccacattccgggtgctactgacatggatac tttggcgttagaatcgccagagacctggaatcgtcggactccagaagaattaaagaaagctt tagaagagcatgggataacggcttcgacgactgtcgttctttatgggaaatttatgcatcccg ataacgcagatgaatttcctggatccgcagctggccatatcggggcgattcgcttggctttca ttatgatgtacgcgggggttgaggacgttcgggtcctgaatggcgggtatcaatcatggacc gatgcgggatttgctatttccaaagatgacgtgccaaagacgactgtcccagagttcggcg cacccattccttcccgtccggaattcgctgtagatatcgacgaagccaaggaaatgcttcaat cagaggattcagatctggtgtgcgtgcgctcgtacccggagtatatcggcgaggtcagcg |
| | | gctataattacataaaaaaaaagggcagaatcccgggggctatatttgctgagtgcgggtc ggatgcctatcacatggaaaactatagaaatcatgaccacaccacgcgcgaatatcatgaa atagaagatatatgggcaaaaagcgggatcatcccgaagaaacacttggctttttattgcgg tactggatggcgtggttctgaggcttggttcaacgcgttgctgatgggttggccgcgcgtttc cgtttatgacggcggttggtttgagtggagcaacgaccccgaaaacccatacgagacggg agttccgaagtga |
| EcHerS | SEQ ID No. 6 | atgtcgggattttcgatgaaacgtgtttctcaaatgaccgcgctggcaatggctttagggctg gcttgcgcttcttcgtgggccgctgaactggcgaagcctcttacacttgaccagcttcaacaa caaaatggcaaagcgattgatactcgccccagcgcgttttataacggctggccacaaacctt aaatggcccttccggtcatgaacctgccgccttaaacctctctgctagctggctcgacaaaat gagcaccgaacagctcaacgagtggatcaagcaacataacctgaaagccgatgccccgg tggcgctgtacggtaatgacaaagatgtcgacgccgtcaaaacgcgactgcaaaaagcag gttttacgcatatctccatcctgagtgacgcgctaagcgaaccttcccgtctgcaaaaactgc cgcactttgaacagctggtatatccgcagtggctgcataacctgcaacaaggtaaagaggtt acggcgaaacctgccggtgactggaaagtcattgaagcggcctggggcgctccaaaatttt accttatcagccatattcccggcgctgactacatcgacaccaacgaagtggaaagcgaacc gctgtggaacaaagtttctgatgaacaactgaaagcgatgctggcaaaacacggcattcgc catgacaccacggtcattctgtacgggcgtgacgtatacgctgcagcgcgtgtggcgcaaa ttatgctttatgccggcgtgaaagatgtgcgcctgctggacggaggctggcaaacctggtc cgacgcgggtctgcccgttgagcgcggaacgccaccgaaagtgaaagcggaaccggatt tcggcgtgaagatcccggcacaaccgcaactgatgcttgatatggaacaagcgcgtggac tgctgcatcgccaggatgcatcgctggtgagcattcgttcgtggccagaatttatcggtacg accagcggttacagctatattaaaccaaaaggtgaaatagccggagcacgttggggacac gcaggtagcgactcgacgcatatggaagatttccataacccggatggcaccatgcgcagc gccgatgatataaccgctatgtggaaagcatggaatatcaaaccagatcagcaagtttcattc tactgcggcactggctggcgcgcgtcagaaacctttatgtacgcacgcgcaatgggctgg aataacgtctccgtttacgacggtggctggtacgaatggagcagcgatccaaaaaatccgg tagcaaccggtgaacgcggcccggacagcagtaaataa |
| CkHerS | -- | atgaccgcgctggcactggcttcaggactcgcctgcgcttcctcctgggctgctgatatggc gcagtcgcttaccttcagccagctacagcaaaaacagggcgccgcgattgatacacgcca gagcgctttttacaacggctggccgcaaacccttaatggcccttccggtcacgaaccttccg cactgaatctgtcagcctcctggctcgataaaatgagcgacgagcagctcgccgcctggct gcaacggcatcagctcaggaaagacgccccgctggcgctgtatggcaatgacaatggcg acaggcggtgaaatcacgtttacagaaggcaggcttcagccagattgctacgctgagcga gcgctgaccgatccttcccgactgcaaaagctccctcacttcgagcaattagcatacccaca |
| | | gtggttgcacgcccttcaacaaaaccagccggttacggctaaaccggcgggagactggaa ggtgattgaagcaggctggggcgcgccgaaatattatctgctcagccacattcccggcgcc ggatacatcgacaccaatgaggtggaaagcgaaccgctgtggaataaagtctctgacgcg cagttgaaagcgatgctggcgaaacacggtattcgccatgacacgacggttattctgtatgg ccgcgacgtatacgctgcggcgcgcgttgcgcagattatgctctatgcgggcgtgaagga cgttcgtctgctcgacggcggctggcaaacctggtccgatgcgggtctgccagttgaacgc ggcatgcccgctgacgtgaagccggaacccgactttggcgtggcgatcccggcgcagcc gcagttaatgctggatatggaacaggcccgcgccctgctgcaccgtcaggacgcatcgct ggtcagcatccgttcctggccggaatttatcggcaccaccagcggatacagctatatcaagc caaaaggcgaaattgccggcgcgcgttggggacatgcgggcagcgactccacgcatatg gaggatttccataacccggacggcaccatgcgcagcgcagaggatatcgccgccatgtgg aagcagtgggatattctgccggatcagcacgttgccttctattgcggcaccggctggcgcg cctccgaaacgtttatgtacgcgcgggcgatgggctggaaaaacgtcgccgtgtatgacg gcggttggtacgaatggagcagcgatccgaaaaatccggtgtccaccggcgagcgcgac ccggacagcagtaagtag |

The present disclosure is further illustrated below in conjunction with examples:

### Example 1 Preparation of MsHisMT enzyme solution

1. The MsHisMT gene fragment was amplified with chromosomal DNA of *Mycobacterium smegmatismc2* 155 (ATCC700084) as a template by PCR using the corresponding primers in Table 1, subjected to enzyme digestion using the Nde I/Xho I purchased from NEB Company, and connected to a pET28a plasmid (purchased from Addgene) digested with the same enzyme. Then the plasmid was transformed into *E coli* DH5a cells (purchased from Tsingke Biotechnology), and verified by colony PCR and gene sequencing.
2. The plasmid with verified correct sequence was transformed into *E. coli* KO1 strains (BL21-DE3 cells in which S-adenosyl homocysteine nucleoside hydrolase mtnN gene was knocked out, wherein the parent strain was obtained from Tsingke Biotechnology, and the gene knockout service was provided by Ubigene Biosciences), which were then cultured in a small scale in 5 ml of LB culture medium containing 50 µM Kanamycin at 37°C. When the cells grew to an OD₆₀₀ value of 0.5-0.8, 0.5 mM isopropyl-β-D-thiogalactopyranoside (IPTG) was added to induce protein expression at 37°C for 3 h. Finally the cells were collected, disrupted by freeze-thaw method, and centrifuged at high speed, and the collected supernatant was subjected to sodium dodecyl sulfate polyacrylamide gel electrophoresis (SDS-PAGE) to confirm the protein expression. After expression in a small scale, MsHisMT was detected to have an enzyme activity of 90-180 U/mg.
3. The strains with correct protein expression were cultured step by step in a 5 liter fermenter to be induced for expression under the condition of 1.0 mM IPTG at 37°C for 4 h, and wet cells were collected to be 30-60 g. Then after the cells and an appropriate amount of Tris.HCl buffer solution (25 mM, pH=8.0) were mixed evenly, the cells were crushed with a high-pressure crusher at low temperature and centrifuged at high speed to remove the cell wall to obtain a MsHisMT enzyme solution, which was stored in a refrigerator at 4°C for later use.

In the above steps, the LB culture medium was composed of 1% tryptone, 0.5% yeast powder, 1% NaCl, 1% dipotassium phosphate, 1% dipotassium phosphate and 5% glycerol.

### Example 2 Preparation of ClHisMT enzyme solution

ClHisMT gene was synthesized by Anhui General Biology Co., Ltd. and subcloned into a pET28a plasmid. The cloning method was the same as step 1 in Example 1, and the restriction site was Nde I/Xho I. The verified correct plasmid was transfected into *E. coli* KO1, cultured in a small scale in 5 ml of LB culture medium to verify the protein expression, and then amplified in a 5-liter fermenter for high-density fermentation. Methods for the small-scale culture and high-density fermentation were the same as steps 2 and 3 in Example 1. After expression in a small scale, ClHisMT was detected to have an enzyme activity of 30-80 U/mg. The bacterial cells after expression in a large amount were disrupted and centrifuged to remove the cell wall to obtain a ClHisMT enzyme solution.

### Example 3 Preparation of CtHaMT enzyme solution

CtHaMT gene was synthesized by Anhui General Biology Co., Ltd. and subcloned into a pET28a plasmid. The cloning method was the same as step 1 in Example 1, and the restriction site was Nde I/Xho I. The verified correct plasmid was transfected into *E. coli* KO1, cultured in a small scale in 5 ml of LB culture medium to verify the protein expression, and then amplified in a 5-liter fermenter for high-density fermentation. Methods for the small-scale culture and high-density fermentation were the same as steps 2 and 3 in Example 1. After expression in a small scale, CtHaMT was detected to have an enzyme activity of 20-60 U/mg. The bacterial cells after expression in a large amount were disrupted and centrifuged to remove the cell wall to obtain a CtHaMT enzyme solution.

### Example 4 Preparation of MspHaMT enzyme solution

MspHaMT gene was synthesized by Anhui General Biology Co., Ltd. and subcloned into a pET28a plasmid. The cloning method was the same as step 1 in Example 1, and the restriction site was Nde I/Xho I. The verified correct plasmid was transfected into *E. coli* KO1, cultured in a small scale in 5 ml of LB culture medium to verify the protein expression, and then amplified in a 5-liter fermenter for high-density fermentation. Methods for the small-scale culture and high-density fermentation were the same as steps 2 and 3 in Example 1. After expression in a small scale, MspHaMT was detected to have an enzyme activity of 15-35 U/mg. The bacterial cells after expression in a large amount were disrupted and centrifuged to remove the cell wall to obtain an MspHaMT enzyme solution.

### Example 5 Preparation of EcHerS enzyme solution

The EcHerS gene fragment was amplified with chromosomal DNA of *Escherichia coli* purchased from ATCC (*Escherichia coli* O6:H1, ATCC 700928) as a template by PCR using the corresponding primers in Table 1, subjected to enzyme digestion using the Nco I/Xho I purchased from NEB Company, and connected to a pET28a plasmid (purchased from Addgene) digested with the same enzyme. Then the plasmid was transformed into *E coli* DH5a cells (purchased from Tsingke Biotechnology), and verified by colony PCR and gene sequencing.

The verified correct plasmid was transfected into *E. coli* KO1, cultured in a small scale in 5 ml of LB culture medium to verify the protein expression, and then amplified in a 5-liter fermenter for high-density fermentation. Methods for the small-scale culture and high-density fermentation were the same as steps 2 and 3 in Example 1. After expression in a small scale, EcHerS was detected to have an enzyme activity of 15-50 U/mg. The bacterial cells after expression in a large amount were disrupted and centrifuged to remove the cell wall. The resulting EcHerS enzyme solution was vacuumized with a diaphragm vacuum pump (Tianjin Jinteng Experimental Equipment Co., Ltd.) for multiple times and supplemented with N₂ to remove O₂ from the reaction solution, and finally sealed under N₂ protection.

### Example 6 Preparation of ClHerS enzyme solution

ClHerS gene fragment was synthesized by Anhui General Biology Co., Ltd. and subcloned into a pET28a plasmid. The cloning method was the same as step 1 in Example 1, and the restriction site was Nde I/Xho I. The verified correct plasmid was transfected into *E. coli* KO1, cultured in a small scale in 5 ml of LB culture medium to verify the protein expression, and then amplified in a 5-liter fermenter for high-density fermentation. Methods for the small-scale culture and high-density fermentation were the same as steps 2 and 3 in Example 1. After expression in a small scale, ClHerS was detected to have an enzyme activity of 8-25 U/mg. The bacterial cells after expression in a large amount were disrupted and centrifuged to remove the cell wall. The resulting EcHerS enzyme solution was vacuumized with a diaphragm vacuum pump (Tianjin Jinteng Experimental Equipment Co., Ltd.) for multiple times and supplemented with N₂ to remove O₂ from the reaction solution, and finally sealed under N₂ protection.

### Example 7: Preparation of L-ergothioneine (combination of MsHisMT, CtHaMT and EcHerS)

### 1. Catalyzing L-histidine to obtain L-hercynine using MsHisMT and CtHaMT:

31 g of L-histidine (200 mM), 0.4 g of S-adenosylmethionine (SAM) (1 mM), 1000 U of MsHisMT and 1500 U of CtHaMT were added to 1 L of 100 mM phosphate buffer solution at pH 8.0. The reaction solution was gently stirred at room temperature, and then slowly added dropwise with 102.2 g of iodomethane (720 mmol) within 4 h. After the dropwise addition was completed, the reaction solution was continuously stirred for 4 h. During the reaction, the reaction system was maintained at pH 7.0-9.0 by adding aqueous solutions of HCl and NaOH at low concentrations dropwise. After the reaction was completed, it was detected by HPLC that most of L-histidine had been converted into L-hercynine (Shimadzu LC-20AT HPLC with Phenomenex Luna^{®} 5 µm SCX 100 Å, LC Column 100×4.6 mm, liquid chromatography conversion rate 94%). The reaction solution was subjected to extraction with 250 ml of ethyl acetate three times to remove iodomethane and iodine followed by ultrafiltration (a molecular cut-off of 1000-5000 molecular weight, Hangzhou Kaijie Membrane Separation Technology Co., Ltd.) to remove enzymes (MsHisMT and CtHaMT) in the reaction system, then vacuumized with a diaphragm vacuum pump and supplemented with N₂ to remove O₂ from the reaction solution, and finally sealed and protected with N₂ for later use. (Activity unit U represents the amount of enzyme required to convert 1 µM substrate per minute at 30°C.)

### 2. Converting L-hercynine to L-ergothioneine using EcHerS:

The above L-hercynine reaction solution was added with 800 U of EcHerS and 12.8 g (twice the equivalent) of potassium polysulfide K₂S_{X} (Sigma-Aldrich), and gently stirred in N₂ atmosphere at room temperature for 6 h. HPLC detection (same as above) showed that most of L-hercynine raw material was consumed. The reaction solution was poured into 3 L of ethanol solution for precipitation, and then centrifuged to remove EcHerS enzyme and phosphate in the reaction solution. The solution was vacuum concentrated to 400 ml, directly loaded on an anion exchange column (Amberjet^{®}4200, -OH type, Sigma-Aldrich), and then eluted with pure water. The collected aqueous samples were concentrated to obtain 52 g of light gray crude product of L-ergothioneine, which was further crystallized in ethanol/water solution to obtain 33.1 g of light yellow solid, with a final total yield of 72%.

### Example 8 Preparation of L-ergothioneine (combination of ClHisMT, CtHaMT, and ClHerS):

### 1. Catalyzing L-histidine to obtain L-hercynine using ClHisMT and CtHaMT:

15.5 g of L-histidine (100 mM), 0.2 g of S-adenosylmethionine (1 mM), 800 U of ClHisMT and 800 U of CtHaMT were added to 1L of 100 mM phosphate buffer solution at pH 8.0. The reaction solution was gently stirred at room temperature (25°C), and then slowly added dropwise with 51.1 g of iodomethane (360 mmol). After the dropwise addition was completed, the reaction solution was continuously stirred for 5 h. When it was detected by HPLC that most of L-histidine had been converted (liquid chromatography conversion rate of 81%), the reaction system was subjected to extraction with 250 ml of ethyl acetate to remove iodomethane and iodine in the reaction system followed by ultrafiltration to remove enzymes (ClHisMT and CtHaMT) in the solution, then deoxygenated, sealed with N₂ (the method was the same as above), and stored at 4°C for later use.

### 2. Converting L-hercynine to L-ergothioneine using ClHerS:

The crude solution of L-hercynine prepared above was added with 600 U of ClHerS and 6.4 g of potassium polysulfide K₂S_{X}, and gently stirred in N₂ atmosphere at room temperature for 6 h. HPLC detection (same as above) showed that most of L-hercynine raw material was consumed. The reaction solution was added to 3 L of ethanol solution to terminate the reaction and precipitate ClHerS enzyme and phosphate, and centrifuged to remove the precipitate. The supernatant was vacuum concentrated to 150 ml with a rotary evaporator. The concentrated reaction solution was purified with an anion exchange column, eluted with pure water (same as above), and concentrated to obtain 41.5 g of a crude product of L-ergothioneine, which was then crystallized with ethanol/water to obtain 14.2 g of whitish solid L-ergothioneine, with a final yield of about 61%.

### Example 9 Preparation of L-ergothioneine (combination of MsHisMT, MspHaMT and ClHerS):

### 1. Catalyzing L-histidine to obtain L-hercynine using MsHisMT and MspHaMT:

Similarly, 15.5 g of L-histidine (100 mM), 0.2 g of S-adenosylmethionine (1 mM), 500 U of MsHisMT and 1000 U of MspHaMT were added to 1 L of 100 mM phosphate buffer solution at pH 8.0. The reaction system was slowly added dropwise with 51.1 g of iodomethane (360 mmole) within about 3 h, and then stirred for about 4 h for reaction. HPLC detection showed that most of L-histidine had been consumed (liquid chromatography conversion rate of 90%). The reaction solution was subjected to extraction with 250 ml of ethyl acetate to remove iodomethane and iodine remained after the reaction followed by ultrafiltration to remove enzymes (MsHisMT and MspHaMT) in the solution, then deoxygenated, sealed with N₂ (the method was the same as above), and stored at 4°C for later use.

### 2. Converting L-hercynine to L-ergothioneine using ClHerS:

Similarly, the above crude solution of L-hercynine was added with 600 U of ClHerS and 6.4 g of potassium polysulfide K₂S_{X}, and gently stirred in N₂ atmosphere at room temperature for 6 h. HPLC detection showed that L-hercynine raw material was completely consumed. The reaction solution was poured into 3 L of ethanol solution to terminate the reaction for precipitation, and centrifuged to remove ClHerS enzyme and phosphate. The resulting solution was concentrated to about 150 ml, and then purified with an Amberjet anion exchange column. The eluate was concentrated to obtain 45 g of a crude product of L-ergothioneine, which was then purified by crystallization with ethanol/water system to obtain 17 g of L-ergothioneine as a light yellow solid, with a final yield about 74%.

### Comparative Example Preparation of L-ergothioneine (combination of ClHisMT, CtHaMT and CkHerS)

The enzyme used in the second step reaction in the preparation of L-ergothioneine is important in the whole preparation system of L-ergothioneine. Most of the HerS enzymes verified in the previous experiments had poor activity and stability, for example, CsHerS derived from *Clostridium scindens* ATCC 35704, CkHerS derived from *Citrobacter koseri* ATCC BAA-895, MsHerS derived from *Mannheimia succiniciproducens,* and LgHerS derived from *Leptotrichia goodfellowii* all had poor activity and stability, resulting in a total yield of only about 10%. The reaction was carried out with CkHerS, which is described in details as follows:

### 1. Preparation of CkHerS enzyme solution

The CkHerS enzyme solution was prepared by the same method as in Example 6. The vector insertion site was Nhe I/Xho I, and the activity of the prepared enzyme was 0.5-7 U/mg.

### 2. Preparation of L-ergothioneine

### 2.1 Catalyzing L-histidine to obtain L-hercynine using ClHisMT and CtHaMT:

15.5 g of L-histidine (100 mM), 0.2 g of S-adenosylmethionine (1 mM), 800 U of ClHisMT and 800 U of CtHaMT were added in 1 L of 100 mM phosphate buffer solution at pH 8.0. The reaction solution was stirred at room temperature (25°C) and slowly added dropwise with 51.1 g of iodomethane (360 mmole). After the dropwise addition was completed, the reaction solution was continuously stirred for 5 h. When it was detected by HPLC that most of L-histidine had been converted (liquid chromatography conversion rate of 79%), the reaction system was subjected to extraction with 250 ml of ethyl acetate to remove iodomethane and iodine in the reaction system followed by ultrafiltration to remove enzymes (ClHisMT and CtHaMT) in the solution, then deoxygenated, sealed with N₂ (the method was the same as above), and stored at 4°C for later use.

### 2.2. Converting L-hercynine to L-ergothioneine using CkHerS:

The crude solution of L-hercynine prepared above was added with 600 U of CkHerS and 6.4 g of potassium polysulfide K₂Sx, and gently stirred in N₂ atmosphere at room temperature for 10 h. HPLC detection (same as above) showed that most of L-hercynine remained. Then the reaction solution was supplemented with 600 U of CkHerS, stirred at room temperature for 20 h, added with 3 L of ethanol to terminate the reaction and precipitate ClHerS enzyme and phosphate, and centrifuged to remove the precipitate. The supernatant was vacuum concentrated to 150 ml with a rotary evaporator. The concentrated reaction solution was purified with an anion exchange column, eluted with pure water (same as above), and concentrated to obtain 43 g of a grey crude product of the reaction solution, which cannot be purified to obtain L-ergothioneine by the above ethanol/water crystallization condition. The final crude product had a HPLC purity of 5.7% and a total yield of the two-step liquid chromatography of 10.5%.

The above are only preferred embodiments of the present disclosure, and it should be noted that for those of ordinary skill in the art, several improvements and modifications can also be made without departing from the principle of the present disclosure, and these improvements and modifications should also be considered as the protection scope of the present disclosure.

## Claims

1. An enzyme composition, comprising L-histidine methyltransferase, halogen methyltransferase and L-hercynine sulfurylase.

2. The enzyme composition according to claim 1, wherein
the L-histidine methyltransferase is derived from *Mycobacterium smegmatismc* or *Chlorobium limicola;*
the halogen methyltransferase is derived from *Chloracidobacterium thermophilum* or *Mycobacterium smegmatis;* and
the L-hercynine sulfurylase is derived from *Escherichia coli* or *Chlorobium limicola.*

3. The enzyme composition according to claim 1 or 2, wherein
the L-histidine methyltransferase has an amino acid sequence set forth in SEQ ID NO: 1 or 2,
the halogen methyltransferase has an amino acid sequence set forth in SEQ ID NO: 3 or 4, and
the L-hercynine sulfurylase has an amino acid sequence set forth in SEQ ID NO:5 or 6.

4. Use of the enzyme composition according to any one of claims 1 to 3 in the production of L-ergothionein by enzymatic catalysis.

5. A method for producing L-ergothionein by enzymatic catalysis, comprising
catalyzing L-histidine by L-histidine methyltransferase and halogen methyltransferase to obtain L-hercynine; and catalyzing L-hercynine by L-hercynine sulfurylase to obtain L-ergothioneine.

6. The method according to claim 5, wherein L-hercynine is prepared by
adding L-histidine, S-adenosylmethionine, L-histidine methyltransferase and halogen methyltransferase to a buffer solution, adding iodomethane dropwise, and stirring at pH7.0-9.0 for reaction to obtain L-hercynine.

7. The method according to claim 6, wherein
the buffer solution is a phosphate buffer solution at 100 mmol/L with a pH of 8.0;
the L-histidine has a concentration of 100 mmol/L-200 mmol/L, the S-adenosylmethionine has a concentration of 0.8 mmol/L-1.2 mmol/L, the L-histidine methyltransferase has a concentration of 500 U/L-1000 U/L, and the halogen methyltransferase has a concentration of 800 U/L-1500 U/L; and
the iodomethane and S-adenosylmethionine are in a molar ratio of (360-720): 1.

8. The method according to claim 5, wherein the catalyzing L-hercynine by L-hercynine sulfurylase to obtain L-ergothioneine comprises adding L-hercynine sulfurylase and potassium polysulfide for reaction in N₂ atmosphere to obtain L-ergothioneine.

9. The method according to claim 8, wherein the L-hercynine sulfurylase has a concentration of 600 U/L-800 U/L, and the potassium polysulfide is added at an amount twice the equivalent of the L-hercynine.

10. The method according to any one of claims 5-9, wherein
the L-histidine methyltransferase is prepared by transforming a host in which S-adenosylhomocysteine nucleoside hydrolase mtnN gene is knocked out with a nucleic acid molecule encoding L-histidine methyltransferase, and performing induction and expression to obtain a bacterial solution containing the L-histidine methyltransferase;
the halogen methyltransferase is prepared by transforming a host in which S-adenosylhomocysteine nucleoside hydrolase mtnN gene is knocked out with a nucleic acid molecule encoding halogen methyltransferase, and performing induction and expression to obtain a bacterial solution containing the halogen methyltransferase; and
the L-hercynine sulfurylase is prepared by transforming a host in which S-adenosylhomocysteine nucleoside hydrolase mtnN gene is knocked out with a nucleic acid molecule encoding L-hercynine sulfurylase, and performing induction and expression to obtain a bacterial solution containing the L-hercynine sulfurylase.

11. A nucleic acid molecule encoding L-histidine methyltransferase, selected from the group consisting of
I) a nucleic acid molecule having a nucleotide sequence set forth in SEQ ID NO: 7 or 8;
II) a nucleic acid molecule derived from I) by substitution, deletion or addition of one or more nucleotides;
III) a nucleic acid molecule having at least 85% homology with I) and encoding L-histidine methyltransferase; and
IV) a nucleic acid molecule partially or fully complementary to any one of I) to III).

12. A nucleic acid molecule encoding halogen methyltransferase, selected from the group consisting of
I) a nucleic acid molecule having a nucleotide sequence set forth in SEQ ID NO: 9 or 10;
II) a nucleic acid molecule derived from I) by substitution, deletion or addition of one or more nucleotides;
III) a nucleic acid molecule having at least 85% homology with I) and encoding L-histidine methyltransferase; and
IV) a nucleic acid molecule partially or fully complementary to any one of I) to III).

13. A nucleic acid molecule encoding L-hercynine sulfurylase, selected from the group consisting of
I) a nucleic acid molecule having a nucleotide sequence set forth in SEQ ID NO: 11 or 12;
II) a nucleic acid molecule derived from I) by substitution, deletion or addition of one or more nucleotides;
III) a nucleic acid molecule having at least 85% homology with I) and encoding L-hercynine sulfurylase; and
IV) a nucleic acid molecule partially or fully complementary to any one of I) to III).

14. A recombinant vector, comprising
the nucleic acid molecule according to claim 11 and a backbone vector;
the nucleic acid molecule according to claim 12 and a backbone vector; or
the nucleic acid molecule according to claim 13 and a backbone vector.

15. A recombinant host transformed or transfected with the recombinant vector according to claim 14, wherein in the host, S-adenosyl homocysteine nucleoside hydrolase mtnN gene is knocked out.
